# EUROPEAN PATENT APPLICATION

(11) **EP 4 056 182 A1**
(43) Date of publication of application: **14.09.2022**
(21) Application number: 20884538.8
(22) Date of filing: 21.10.2020
(51) Int. Cl.: A61K 31/513, A61P 9/12

(54) **CRYSTAL FORM OF APROCITENTAN, PREPARATION METHOD THEREFOR AND USE THEREOF**

(30) Priority: 07.11.2019 CN 201911073945
(71) Applicant: Crystal Pharmaceutical (Suzhou) Co., Ltd., Suzhou, Jiangsu 215123 (CN)
(72) Inventor: CHEN, Minhua, Suzhou, Jiangsu 215123 (CN); ZHU, Hongyan, Suzhou, Jiangsu 215123 (CN)
(74) Representative: dompatent von Kreisler Selting Werner - Partnerschaft von Patent- und Rechtsanwälten mbB
(86) International application number: PCT/CN2020/122546
(87) International publication number: WO 2021/088645

(57) **Abstract**

The present disclosure relates to a novel crystalline form of Aprocitentan (hereinafter referred to as "Compound I") and preparation methods thereof, pharmaceutical compositions containing the crystalline form, and uses of the crystalline form for preparing dual endothelin receptor antagonist drugs and drugs for treating resistant hypertension. Compared with prior arts, the provided crystalline form of Compound I has one or more improved properties and has significant value for future drug optimization and development.

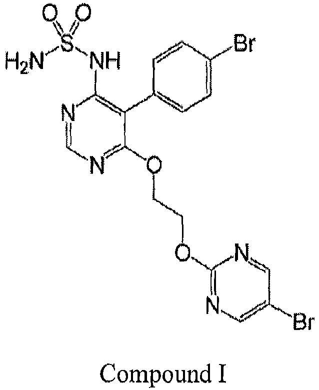

## Description

### TECHNICAL FIELD

The present disclosure pertains to the field of chemical crystallography, particularly relates to crystalline form of Aprocitentan, preparation method and use thereof.

### BACKGROUND

Resistant hypertension (RH) is the main cause of heart failure, chronic renal failure and multiple cardiovascular diseases. RH is defined as a blood pressure that remains above goal despite concurrent use of three antihypertensive agents taken at maximally tolerated doses, one of which should be a diuretic. It means that patients with RH require four or more medications to control the blood pressure.

Aprocitentan, also known as ACT-132577, is a dual endothelin receptor antagonist and is being developed by Idorsia Pharmaceuticals for the treatment of diseases related to vasoconstriction, proliferation or inflammation caused by the increase of endothelin level, such as hypertension, pulmonary hypertension, coronary heart disease, heart failure, renal and myocardial ischemia, renal failure, etc. The results of the phase II clinical trial suggest that Aprocitentan has achieved good effects in the treatment of resistant hypertension.

The chemical name of Aprocitentan is {5-(4-bromo-phenyl)-6-[2-(5-bromo-pyrimidin-2-yloxy)-ethoxy]-pyrimidin-4-yl}-sulfamide (hereinafter referred to as "Compound I"), and the structure is shown as follows:

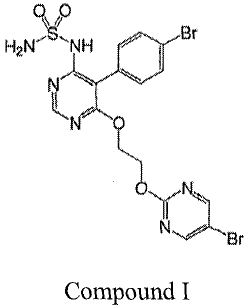

A crystalline form is a solid material whose constituents are arranged in a highly ordered microscopic structure, forming a crystal lattice that extends in all directions. Polymorphism is the ability of a compound to exist in two or more than two crystalline forms. Different crystalline forms have different physicochemical properties and can affect drug's in vivo dissolution and absorption, which will further affect drug's clinical efficacy and safety to some extent. In particular, for poorly soluble drugs, the above effects of the crystalline form will be greater. Therefore, drug polymorphism is an important part of drug research and an important part of drug quality control.

WO2018154101A1 disclosed crystalline forms A, B, C, D, E, J, K, L and amorphous of Compound I free form. According to the description of WO2018154101A1, the properties of the above crystalline forms are shown in Table 1 below.

**Table 1**

| Crystalline form | Properties of crystalline forms disclosed in prior art |
|---|---|
| Form A | Form A is an anhydrate or non- solvate form with a melting point of 159 °C, and is not the most thermodynamically stable form |
| Form B | Dichloromethane solvate |
| Form C | Form C is an anhydrate or non- solvate form with a melting point of 153 °C, and is slightly hygroscopic |
| Form D | No other disclosure but XRPD |
| Form E | Acetonitrile solvate |
| Form J | No other disclosure but XRPD |
| Form K | Dimethyl sulfoxide (DMSO) solvate |
| Form L | Ethanol solvate |

The description of WO2018154101A1 showed that Form B, Form E, Form K and Form L are not suitable for medicinal use as containing toxic organic solvents. Although Form C is an anhydrate or non- solvate form, it has a certain degree of hygroscopicity, which is not conducive to storage. Form A is an anhydrate or non-solvate form with better properties compared with other crystalline forms disclosed in prior art. When preparing tablets containing Compound I, Form A with better properties is preferred.

Among all the crystalline forms of Compound I disclosed in prior art, Form A is the most suitable form for pharmaceutical development. However, the preparation process of Form A disclosed in WO2018154101A1 is very complicated, which comprise crystallizing Compound I in a pH 8.5-8.0 organic solvent to obtain a crude product of Form A and Form K mixture, washing the crude product multiple times, stirring the slurry for a long time, washing again and drying to obtain Form A product. The inventors of the present disclosure repeated the preparation method disclosed in WO2018154101A1 to obtain Form A and the properties were characterized accordingly. It was found that Form A has poor stability as almost all Form A transforms into amorphous after grinding. Therefore, a crystalline form which is easy to prepare, of good stability, safe and non-toxic, is still needed for the development of drugs containing Compound I.

In order to overcome the disadvantages of prior arts, the inventors of the present disclosure have conducted a large number of experiments on Compound I to try to obtain a crystalline form that is more suitable for medicinal use, but most of the crystalline forms obtained are solvates, such as 1,4-dioxane solvate, methyl tert-butyl ether solvate, trichloromethane solvate, anisole solvate, dimethylformamide solvate. Considering the disclosure of prior art WO2018154101A1, it can be seen that Compound I is prone to form solvates, while a non- form of Compound I is difficult to prepare.

The inventors of the present disclosure did a lot of creative work, and surprisingly discovered crystalline form CSI, which has advantages in physiochemical properties, formulation processability and bioavailability. For example, crystalline form CSI has advantages in at least one aspect of melting point, solubility, hygroscopicity, purification ability, stability, adhesiveness, compressibility, flowability, in vitro and in vivo dissolution, and bioavailability, etc. In particular, crystalline form CSI has low hygroscopicity, good thermodynamic stability, good physical stability, good mechanical stability, uniform particle size distribution, large density and good formulation stability, which provides a new and better choice for the development of Compound I and is of great significance.

### SUMMARY

The main objective of the present disclosure is to provide a novel crystalline form of Compound I, preparation method and use thereof.

According to the objective of the present disclosure, crystalline form CSI of Compound I is provided (hereinafter referred to as Form CSI).

In one aspect provided herein, the X-ray powder diffraction pattern of Form CSI comprises characteristic peaks at 2theta values of 14.2°±0.2°, 21.1°±0.2°and 23.5°±0.2° using CuKa radiation.

Furthermore, the X-ray powder diffraction pattern of Form CSI comprises one or two or three characteristic peaks at 2theta values of 19.9°±0.2°, 28.8°±0.2° and 27.3°±0.2° using CuKa radiation. Preferably, the X-ray powder diffraction pattern of Form CSI comprises three characteristic peaks at 2theta values of 19.9°±0.2°, 28.8°±0.2° and 27.3°±0.2° using CuKa radiation.

Furthermore, the X-ray powder diffraction pattern of Form CSI comprises one or two or three characteristic peaks at 2theta values of 25.9°±0.2°, 28.0°±0.2° and 29.6°±0.2° using CuKa radiation. Preferably, the X-ray powder diffraction pattern of Form CSI comprises characteristic peaks at 2theta values of 25.9°±0.2°, 28.0°±0.2° and 29.6°±0.2° using CuKa radiation.

In another aspect provided herein, the X-ray powder diffraction pattern of Form CSI comprises three or four or five or six or seven or eight or nine or ten or eleven characteristic peaks at 2theta values of 13.5°±0.2°, 14.2°±0.2°, 21.1°±0.2°, 23.5°±0.2°, 19.9°±0.2°, 28.8°±0.2°, 27.3°±0.2°, 25.9°±0.2°, 28.0°±0.2°, 29.6°±0.2° and 27.0°±0.2° using CuKa radiation.

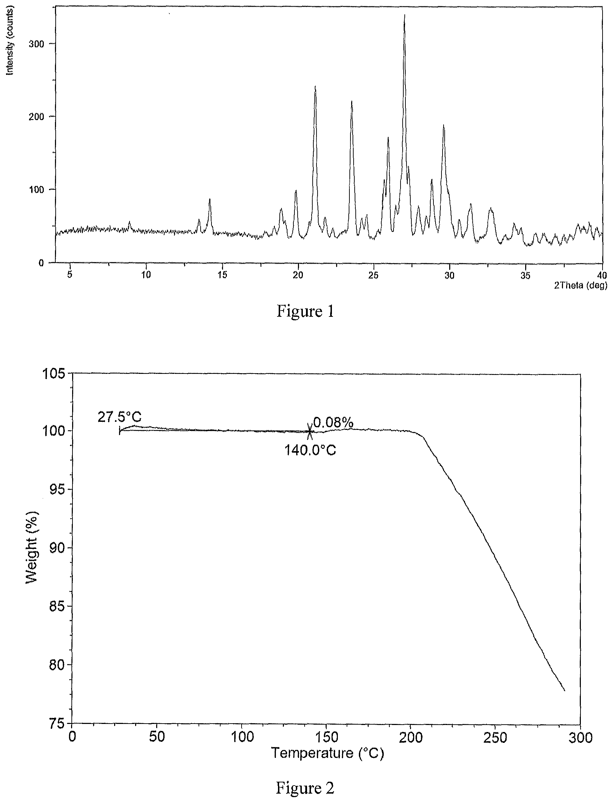

Without any limitation being implied, the X-ray powder diffraction pattern of Form CSI is substantially as depicted in Figure 1.

Without any limitation being implied, the Thermo Gravimetric Analysis (TGA) curve of Form CSI is substantially as depicted in Figure 2, which shows 0.1% weight loss when heated to 140 °C.

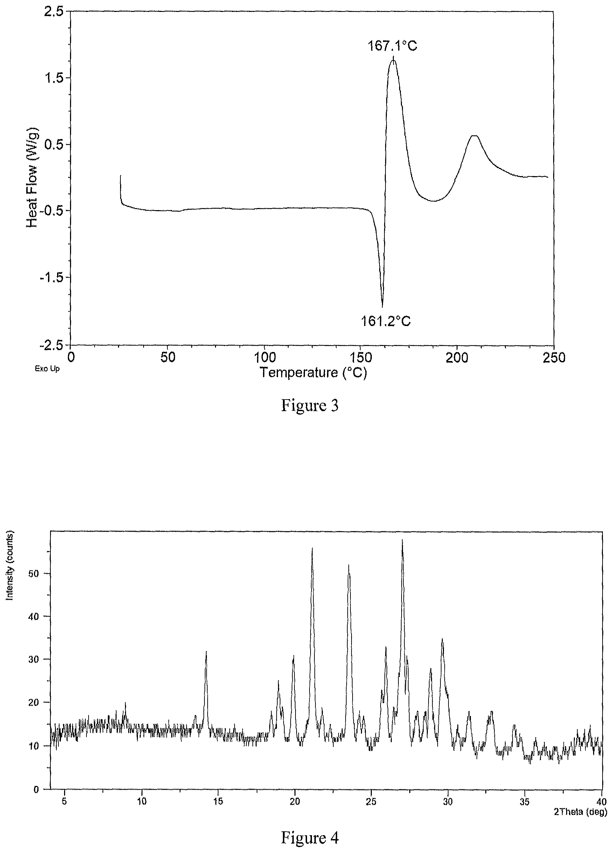

Without any limitation being implied, the Differential Scanning Calorimetry (DSC) curve of Form CSI is substantially as depicted in Figure 3, which shows an endothermic peak at around 161 °C corresponding to the melting process, and shows an exothermic peak at around 167 °C, corresponding to the decomposition process.

Without any limitation being implied, Form CSI is an anhydrate.

According to the objective of the present disclosure, a process for preparing Form CSI is also provided. The process comprises:
(1) adding Compound I solid in a mixture of water and an ester, stirring the suspension, centrifuging and drying the solid to obtain Form CSI; or
(2) dispersing Compound I solid and Form CSI as seed in an alcohol, an ester or a mixture of water and an ester, stirring the suspension, centrifuging and drying to obtain Form CSI.

Furthermore, in method (1), said ester is preferably ethyl acetate; the ratio of water and ethyl acetate of the mixture is preferably 1:20-1:3, more preferably 1:9; said stirring temperature is preferably -15 °C-15 °C, more preferably 5 °C; the time of said stirring is preferably more than 10 days, more preferably 15 days. In method (2), said alcohol is preferably ethanol, said ester is preferably ethyl acetate, said stirring temperature is preferably -20 °C-70 °C, the time of said stirring is preferably more than half an hour.

Form CSI of the present disclosure has the following advantages:
(1) Compared with prior art, Form CSI drug substance of the present disclosure has better stability itself and in drug product. Crystalline state of Form CSI drug substance doesn't change for at least six months when stored under the condition of 25 °C/60%RH (Relative humidity) (open and sealed), and the chemical purity is above 99.8%. After Form CSI is mixed with excipients to form a drug product and stored under the condition of 25 °C/60% RH, crystalline state of Form CSI in the drug product doesn't change for at least one month. The chemical purity is above 99.8%. These results show that Form CSI drug substance of the present disclosure has good stability under long term condition both itself and in drug product, which is beneficial to the storage of the drug.

Meanwhile, crystalline state of Form CSI drug substance doesn't change for at least six months when stored under the condition of 40 °C/75%RH (open and sealed). The chemical purity remains substantially unchanged during storage. Form CSI drug substance doesn't change for at least two months when stored under the condition of 60 °C/75%RH (open and sealed). Prior art Form A shows an obvious crystal transformation after stored at 60 °C/75%RH (open) for one month. After Form CSI is mixed with excipients to form a drug product and stored under the condition of 40 °C/75%RH, crystalline state of Form CSI in the drug product doesn't change for at least one month. The chemical purity is above 99.8% and remains substantially unchanged during storage. These results show that Form CSI drug substance has better stability under accelerated and stress conditions both itself and in drug product. Drug substance and drug product will go through high temperature and high humidity conditions caused by different season, regional climate and weather during storage, transportation, and manufacturing processes. Therefore, good stability under accelerated and stress conditions is of great importance to the drug development. Form CSI drug substance has good stability under stress conditions both itself and in drug product, which is beneficial to avoid the influence on drug quality when not stored in condition recommended in label.

Meanwhile, Form CSI has good mechanical stability. The crystalline state of Form CSI doesn't change after grinding, which has good physical stability. Grinding and pulverization are often required in the drug manufacturing process. Good physical stability of the drug substance can reduce the risk of crystallinity decrease and crystal transformation during the drug production process. Form CSI has good physical stability under different pressure, which is beneficial to keep crystalline form unchanged during tableting process.

Crystal transformation can lead to changes in the absorption of the drug, affect bioavailability, and even cause toxicity and side effects. Form CSI has good physical and chemical stability, ensuring consistent and controllable quality of the drug substance and drug product, minimizing quality changes, bioavailability changes, toxicity and side effects caused by crystal transformation or impurity generation.

(2) Compared with prior art, Form CSI has better thermodynamic stability. When mixtures of Form CSI and prior art Form A are suspended in different solvent systems and stirred at different temperatures, Form CSI is always obtained ultimately, which indicates that Form CSI is thermodynamically more stable.

(3) Compared with prior art, Form CSI of the present disclosure has lower hygroscopicity. The test results show that the weight gain of Form CSI at 80% RH is 0.04% indicating that Form CSI is non hygroscopic or almost non hygroscopic.

Hygroscopicity affects the physicochemical stability of the drug directly, as high hygroscopicity tends to cause chemical degradation and polymorph transformation. In addition, high hygroscopicity will reduce the flowability of the drug, thereby affecting the processing of the drug. Moreover, drug substances with high hygroscopicity require low humidity environment during production and storage, which puts strict requirements on production and imposes higher costs. More importantly, high hygroscopicity is likely to cause variation in the content of active pharmaceutical ingredients in the drug, thus affecting drug quality. The crystalline form with low hygroscopicity is not demanding on the environment, which reduces the cost of production, storage and quality control, and has strong economic value.

(4) Compared with prior art, Form CSI of the present disclosure has higher density. Test results indicate that the bulk density and tapped density of Form CSI are higher than that of prior art form. Higher density of Form CSI is beneficial to large scale production. Higher density of Form CSI can also reduce dust, reduce occupational hazard and ensure production safety.

(5) Compared with prior art, Form CSI of the present disclosure shows superior adhesiveness. Adhesiveness evaluation results indicate that average adhesion quantity of Form CSI is lower than that of prior art Form A. Due to the superior adhesiveness of Form CSI, adhesion to roller and tooling during dry-granulation and compression process can be reduced or avoided, which is also beneficial to improve product appearance and weight variation. In addition, superior adhesiveness of Form CSI can reduce the agglomeration of drug substance, which is beneficial to the dispersion of drug substance, reduces the adhesion between drug substance and instruments, and improves the blend uniformity and content uniformity of drug product.

(6) Form CSI of the present disclosure has uniform particle size distribution. Uniform particle size of Form CSI helps to ensure uniformity of content and reduce variability of in vitro dissolution. At the same time, the preparation process can be simplified, the pretreatment of the drug substance is not required, the cost is reduced, and the risk of decrease in crystallinity and crystal transformation caused by grinding can be reduced. According to the objective of the present disclosure, a pharmaceutical composition is provided, said pharmaceutical composition comprises a therapeutically effective amount of Form CSI and pharmaceutically acceptable carriers or excipients.

Furthermore, Form CSI can be used for preparing drugs antagonizing dual endothelin receptor.

Furthermore, Form CSI can be used for preparing drugs treating resistant hypertension.

In the present disclosure, said "stirring" is accomplished by using a conventional method in the field such as magnetic stirring or mechanical stirring and the stirring speed is 50 to 1800 r/min, preferably the magnetic stirring speed is 300 to 900 r/min and mechanical stirring speed is 100 to 300 r/min.

Said "separation" is accomplished by using a conventional method in the field such as centrifugation or filtration. The operation of "centrifugation" is as follows: the sample to be separated is placed into a centrifuge tube, and then centrifuged at a rate of 10000 r/min until the solid all sink to the bottom of the tube.

Said "drying" is accomplished at room temperature or a higher temperature. The drying temperature is from room temperature to about 80 °C, or to 60 °C, or to 40 °C. The drying time can be more than half an hour, or overnight. Drying is accomplished in a fume hood, a forced air convection oven or a vacuum oven.

In the present disclosure, "crystal" or "crystalline form" refers to the crystal or the crystalline form being identified by the X-ray diffraction pattern shown herein. Those skilled in the art are able to understand that physicochemical properties discussed herein can be characterized. The experimental errors depend on the instrument conditions, the sample preparation and the purity of samples. In particular, those skilled in the art generally know that the X-ray diffraction pattern typically varies with the test conditions and parameters of the instrument, and the model of the instrument. It is necessary to point out that, the relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. In addition, the experimental error of the diffraction peak position is usually 5% or less, and the error of these positions should also be taken into account. An error of ±0.2° is usually allowed. In addition, due to experimental factors such as sample thickness, the overall offset of the diffraction peak is caused, and a certain offset is usually allowed. Thus, it will be understood by those skilled in the art that a crystalline form of the present disclosure is not necessarily to have the exact same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSI of the present disclosure is pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

### BRIEF DESCRIPTION OF THE DRAWINGS

Figure 1 shows an XRPD pattern of Form CSI in Example 1
Figure 2 shows a TGA curve of Form CSI in Example 1
Figure 3 shows a DSC curve of Form CSI in Example 1
Figure 4 shows an XRPD pattern of Form CSI in Example 2
Figure 5 shows an XRPD pattern of Form CSI in Example 3
Figure 6 shows an XRPD pattern of Form CSI in Example 4
Figure 7 shows a ¹H NMR spectrum of Form CSI in Example 4
Figure 8 shows an XRPD pattern overlay of Form CSI stored under different conditions (from top to bottom: initial, stored at 25 °C/60%RH (sealed) for six months, stored at 25 °C/60%RH (open) for six months, stored at 40 °C/75%RH (sealed) for six months, stored at 40 °C/75%RH (open) for six months, stored at 60 °C/75%RH (sealed) for two months, stored at 60 °C/75%RH (open) for two months)
Figure 9 shows an XRPD pattern overlay of Form A stored at 60 °C/75%RH (open) for one month (top: initial, bottom: after storage)
Figure 10 shows an XRPD pattern overlay of mixture of Form CSI and Form A stirred at room temperature (from top to bottom: initial Form A, initial Form CSI, mixture of Form CSI and Form A stirred for 2 days)
Figure 11 shows an XRPD pattern overlay of Form CSI after tableting under different pressures (from top to bottom: initial, tableting under 3 kN, tableting under 7 kN, tableting under 14 kN)
Figure 12 shows an XRPD pattern overlay of Form CSI before and after grinding (top: after grinding, bottom: before grinding)
Figure 13 shows an XRPD pattern overlay of Form A before and after grinding (top: after grinding, bottom: before grinding)
Figure 14 shows a DVS plot of Form CSI
Figure 15 shows an XRPD pattern overlay of Form CSI before and after formulation process (from top to bottom: excipients, after formulation process, and before formulation process)
Figure 16 shows an XRPD pattern overlay of Form CSI drug product stored under different conditions for one month (from top to bottom: excipients, initial drug product, stored under 25 °C/60%RH, stored under 40 °C/75%RH)

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
XRPD: X-ray Powder Diffraction
DSC: Differential Scanning Calorimetry
TGA: Thermo Gravimetric Analysis
DVS: Dynamic Vapor Sorption
PSD: Particle Size Distribution
¹H NMR: Proton Nuclear Magnetic Resonance
HPLC: High Performance Liquid Chromatography

Instruments and methods used for data collection:
X-ray powder diffraction patterns in the present disclosure examples 1-3, 5-6, 11-13 were acquired by a Bruker D8 DISCOVER X-ray powder diffractometer. The parameters of the X-ray powder diffraction method of the present disclosure are as follows:
   X-ray source: Cu, Kα
   Kα1 (Å): 1.54060; Kα2 (Å): 1.54439
   Kα2/Kα1 intensity ratio: 0.50
   Voltage: 40 (kV)
   Current: 40 (mA)
   Scan range: from 4.0 degree to 40.0 degree
X-ray powder diffraction patterns in the present disclosure examples 4 and 7 were acquired by a Bruker D2 PHASER X-ray powder diffractometer. The parameters of the X-ray powder diffraction method of the present disclosure are as follows:
   X-ray source: Cu, Kα
   Kα1 (Å): 1.54060; Kα2 (Å): 1.54439
   Kα2/Kα1 intensity ratio: 0.50
   Voltage: 30 (kV)
   Current: 10 (mA)
   Scan range: from 3.0 degree to 40.0 degree
Differential scanning calorimetry (DSC) data in the present disclosure were acquired by a TA Q2000. The parameters of the DSC method of the present disclosure are as follows:
   Heating rate: 10 °C/min
   Purge gas: nitrogen
Thermo gravimetric analysis (TGA) data in the present disclosure were acquired by a TA Q500. The parameters of the TGA method of the present disclosure are as follows:
   Heating rate: 10 °C/ min
   Purge gas: nitrogen
Dynamic Vapor Sorption (DVS) was measured via an SMS (Surface Measurement Systems Ltd.) intrinsic DVS instrument. Its control software was DVS- Intrinsic control software, and its analysis software was DVS-Intrinsic Analysis software. Typical Parameters for DVS test are as follows:
   Temperature: 25 °C
   Gas and flow rate: N₂, 200 mL/min
   Relative humidity range: 0% RH to 95% RH

The particle size distribution data in the present disclosure were acquired by a Mastersizer 3000 laser particle size analyzer of Malvern. The test was carried out in wet mode, Hydro MV disperser was used for wet test, and the dispersion medium was Isopar G. The parameters are shown in Table 2.

**Table 2**

| Size distribution: Volume | Run Time: 10 s |
|---|---|
| Dispersion medium: Isopar G | Particle coordinates: Standard |
| Run Number: Average of 3 runs | Fluid refractive index: 1.42 |
| Absorptivity: 0.100 | Residuals: Enabled |
| Particle refractive index: 1.520 | Rotation rate: 2000 rpm |
| Particle shape: Irregular | Ultrasonication power/ultrasonication time: 30 W/30 s |

| | |
|---|---|
| ¹H NMR were collected from a Bruker Avance II DMX 400M HZ NMR spectrometer. 1-5 mg of sample was weighed and dissolved in 0.5 mL of deuterated chloroform to obtain a solution with a concentration of 2-10 mg/mL. | |

The parameters for purity test of HPLC in the present disclosure are shown in Table 3.

**Table 3**

| | | |
|---|---|---|
| HPLC | Agilent 1260 with VWD/DAD detector | |
| Column | Waters XBridge Shield RP18,4.6 mm^{∗}150 mm,3.5 µm | |
| Mobile Phase | A: 10 mmol/L K₂HPO₄ aqueous solution | |
| | B: Acetonitrile | |

| | Time (min) | % B |
|---|---|---|
| Gradient | 0.0 | 5 |
| | 0.5 | 5 |
| | 30.0 | 80 |
| | 35.0 | 80 |
| | 36.1 | 5 |
| | 40.0 | 5 |
| Running time | 40.0 min | |
| Equilibrium time | 0.0 min | |
| Flow rate | 1.0 mL/min | |
| Injection Volume | 5 µL | |
| Detection wavelength | UV at 230 nm | |
| Column Temperature | 40 °C | |
| Sample Temperature | Room Temperature | |
| Diluent | Acetonitrile | |

Unless otherwise specified, the following examples were conducted at room temperature. Said "room temperature" is not a specific temperature, but a temperature range of 10-30 °C.

According to the present disclosure, Compound I and/or its salt used as a raw material includes but not limited to solid (crystalline or amorphous), oil, liquid and solution. Preferably, Compound I and/ or its salts and/ or its acetonitrile solvate used as a raw material are solid.

Compound I free base solid used in the following examples can be prepared by known methods in the prior art, for example, the method disclosed in WO2018154101A1.

### Examples

### Examples 1-4: Preparation of Form CSI

### Example 1

1146.2 mg of Compound I was added into 20 mL of tetrahydrofuran to form a clear solution. The clear solution was filtered, and rotary evaporated at 45 °C to obtain a dry amorphous solid. 35.8 mg of the amorphous solid was added into 0.3 mL of water/ethyl acetate (1:9, v/v) and the suspension was stirred at 5 °C for 18 days. The suspension was centrifuged and the obtained solid was dried at room temperature to obtain a white crystalline solid.

The crystalline solid was confirmed to be Form CSI of the present disclosure.

The XRPD pattern of Form CSI obtained in the present example is substantially as depicted in Figure 1, and the XRPD data are listed in Table 4.

The TGA curve shows about 0.1% weight loss when heated to 140 °C, which is substantially as depicted in Figure 2.

The DSC curve is substantially as depicted in Figure 3, which shows an endothermic peak at around 161 °C corresponding to the melting process, and an exothermic peak at around 167 °C corresponding to the decomposition process.

**Table 4**

| 2θ | d spacing | Intensity % |
|---|---|---|
| 8.89 | 9.95 | 3.04 |
| 13.44 | 6.59 | 5.51 |
| 14.14 | 6.26 | 15.08 |
| 18.81 | 4.72 | 12.32 |
| 19.82 | 4.48 | 20.67 |
| 21.12 | 4.21 | 62.80 |
| 21.73 | 4.09 | 9.31 |
| 23.50 | 3.79 | 60.33 |
| 24.17 | 3.68 | 9.42 |
| 24.52 | 3.63 | 11.08 |
| 25.64 | 3.47 | 26.12 |
| 25.91 | 3.44 | 45.70 |
| 26.40 | 3.38 | 15.22 |
| 27.00 | 3.30 | 100.00 |
| 27.27 | 3.27 | 33.13 |
| 27.92 | 3.20 | 15.32 |
| 28.43 | 3.14 | 11.01 |
| 28.76 | 3.10 | 25.71 |
| 29.59 | 3.02 | 51.88 |
| 30.59 | 2.92 | 10.12 |
| 31.38 | 2.85 | 17.22 |
| 32.63 | 2.74 | 14.63 |
| 32.87 | 2.72 | 12.98 |
| 34.19 | 2.62 | 8.29 |
| 34.70 | 2.59 | 7.44 |
| 35.63 | 2.52 | 4.88 |
| 36.17 | 2.48 | 4.66 |
| 36.97 | 2.43 | 3.34 |
| 38.37 | 2.35 | 8.70 |
| 39.17 | 2.30 | 8.62 |
| 39.58 | 2.28 | 6.85 |

### Example 2

11.2 mg of amorphous Compound I obtained in Example 1 was added into 0.3 mL of water/ethyl acetate (1:9, v/v). The suspension was stirred at 4 °C for 12 days, and then centrifuged. The obtained solid was dried naturally at room temperature to obtain a white crystalline solid. The crystalline solid was confirmed to be Form CSI of the present disclosure.

The XRPD pattern of Form CSI obtained in the present example is substantially as depicted in Figure 4, and the XRPD data are listed in Table 5.

**Table 5**

| 2θ | d spacing | Intensity % |
|---|---|---|
| 8.94 | 9.89 | 7.49 |
| 13.49 | 6.56 | 8.17 |
| 14.16 | 6.25 | 35.52 |
| 18.41 | 4.82 | 12.17 |
| 18.90 | 4.70 | 25.56 |
| 19.17 | 4.63 | 15.82 |
| 19.93 | 4.45 | 37.93 |
| 21.11 | 4.21 | 91.88 |
| 21.78 | 4.08 | 16.55 |
| 23.50 | 3.79 | 82.47 |
| 24.21 | 3.68 | 14.68 |
| 24.53 | 3.63 | 13.78 |
| 25.68 | 3.47 | 25.56 |
| 25.94 | 3.43 | 48.81 |
| 27.00 | 3.30 | 100.00 |
| 27.33 | 3.26 | 41.70 |
| 28.00 | 3.19 | 18.61 |
| 28.49 | 3.13 | 17.87 |
| 28.92 | 3.09 | 34.78 |
| 29.59 | 3.02 | 51.95 |
| 30.64 | 2.92 | 12.23 |
| 31.43 | 2.85 | 17.25 |
| 32.84 | 2.73 | 21.15 |
| 34.28 | 2.62 | 15.61 |
| 34.75 | 2.58 | 8.75 |
| 35.69 | 2.52 | 8.07 |
| 36.97 | 2.43 | 4.28 |
| 38.39 | 2.34 | 9.96 |
| 39.19 | 2.30 | 11.84 |
| 39.72 | 2.27 | 8.95 |

### Example 3

9.3 mg of acetonitrile solvate Form E of Compound I (prepared by the method in WO2018154101A1) was added into 0.3 mL of water/ethyl acetate (1:9, v/v). The suspension was stirred at 4 °C for 12 days, and then centrifuged. The obtained solid was dried naturally at room temperature to obtain a white crystalline solid. The crystalline solid was confirmed to be Form CSI of the present disclosure.

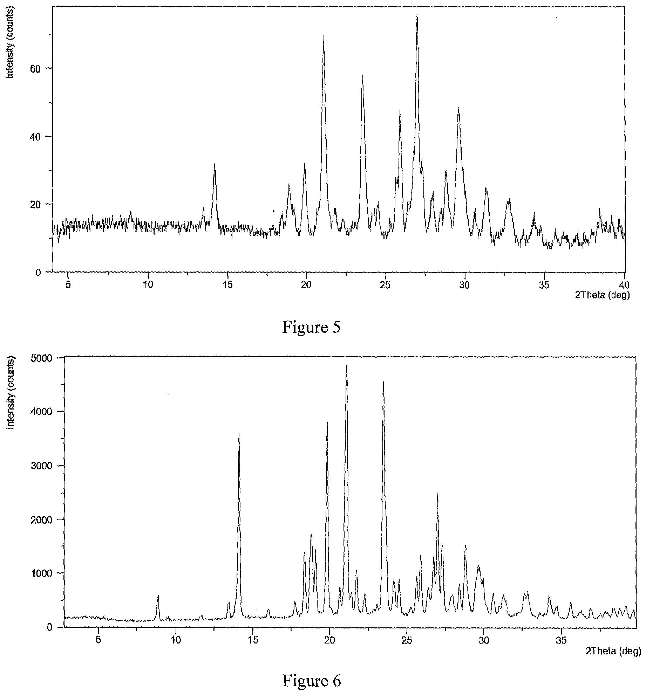

The XRPD pattern of Form CSI obtained in the present example is substantially as depicted in Figure 5, and the XRPD data are listed in Table 6.

**Table 6**

| 2θ | d spacing | Intensity % |
|---|---|---|
| 8.89 | 9.95 | 5.79 |
| 13.51 | 6.55 | 10.06 |
| 14.21 | 6.23 | 28.43 |
| 18.51 | 4.79 | 7.78 |
| 18.97 | 4.68 | 19.43 |
| 19.93 | 4.46 | 30.54 |
| 21.13 | 4.21 | 88.09 |
| 21.86 | 4.07 | 10.80 |
| 22.32 | 3.98 | 6.81 |
| 23.50 | 3.79 | 67.49 |
| 24.23 | 3.67 | 11.05 |
| 24.49 | 3.63 | 14.14 |
| 25.65 | 3.47 | 26.85 |
| 25.92 | 3.44 | 57.75 |
| 26.39 | 3.38 | 15.62 |
| 27.00 | 3.30 | 100.00 |
| 27.10 | 3.30 | 73.12 |
| 27.36 | 3.26 | 30.05 |
| 27.97 | 3.19 | 19.85 |
| 28.47 | 3.13 | 12.41 |
| 28.81 | 3.10 | 31.43 |
| 29.58 | 3.02 | 60.09 |
| 30.66 | 2.91 | 14.18 |
| 31.37 | 2.85 | 22.84 |
| 31.98 | 2.80 | 3.71 |
| 32.66 | 2.74 | 17.47 |
| 33.69 | 2.66 | 5.40 |
| 34.39 | 2.61 | 11.47 |
| 35.68 | 2.51 | 4.54 |
| 36.29 | 2.47 | 3.26 |
| 37.01 | 2.43 | 3.21 |
| 38.46 | 2.34 | 12.94 |
| 39.23 | 2.29 | 9.78 |
| 39.63 | 2.27 | 9.07 |

### Example 4

As shown in Table 7, certain amount of Compound I solid was weighed. A certain amount of Form CSI seed and a certain volume of solvent were added. These suspensions were stirred for a certain period of time, centrifuged, and dried to obtain white crystalline solids. The obtained solids were labeled as samples 1-10. Samples 1-10 were all confirmed to be Form CSI. The XRPD pattern of Form CSI is substantially as depicted in Figure 6.

**Table 7**

| No. | Mass of Compound I(mg) | Solvent volume (mL) | Solvent (v: v) | Seed (mg) | Stirring time (day) | Stirring temperature | Form |
|---|---|---|---|---|---|---|---|
| 1 | 41.9 | 0.3 | water/ethyl acetate (1:9) | 1.8 | 11 | 5 °C | Form CSI |
| 2 | 53.3 | 0.3 | water/ethyl acetate (1:9) | 2.0 | 11 | Room temperature | |
| 3 | 82.4 | 0.5 | water/ethyl acetate (1:9) | 10 | 3 | Room temperature | |
| 4 | 518.4 | 3 | water/ethyl acetate (1:9) | 10 | 3 | Room temperature | |
| 5 | 1338.1 | 7 | water/ethyl acetate (1:9) | 10 | 3 | Room temperature | |
| 6 | 18.2 | 0.25 | ethanol | 4.1 | 2 | 50 °C | |
| 7 | 15.0 | 0.25 | ethanol | 3.5 | 2 | Room temperature | |
| 8 | 24.1 | 0.25 | ethyl acetate | 4.6 | 2 | 50 °C | |
| 9 | 12.6 | 0.25 | ethyl acetate | 5.3 | 2 | Room temperature | |
| 10 | 14.1 | 0.3 | water/ethyl acetate (1:9) | 8.8 | 2 | Room temperature | |

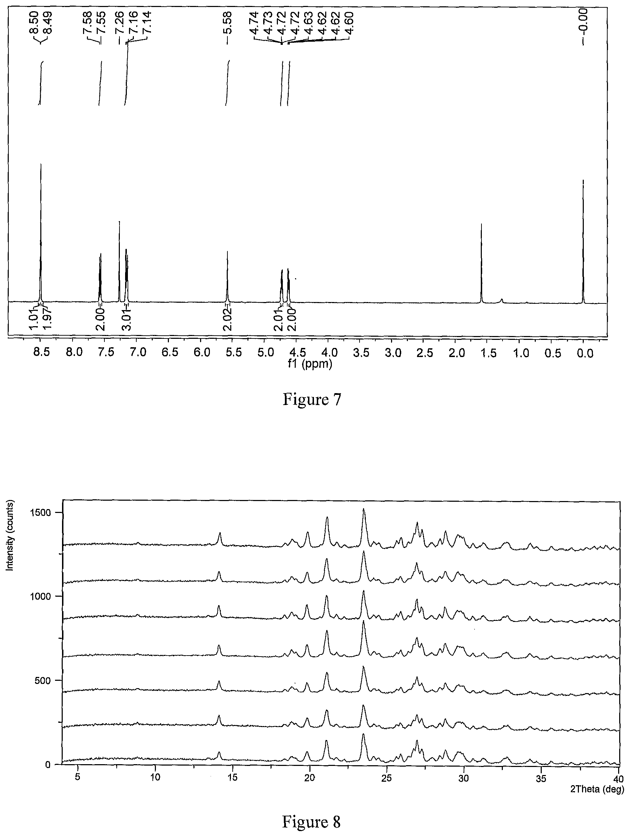

The ¹H NMR spectrum of Form CSI is substantially as depicted in Figure 7, and the corresponding data are: ¹H NMR (400 MHz, CDCl₃) δ 8.50 (s, 1H), 8.49 (s, 2H), 7.67-7.44 (m, 2H), 7.19-7.11 (m, 3H), 5.58 (s, 2H), 4.74-4.72 (m, 2H), 4.63-4.60 (m, 2H).

### Example 5 Physical and chemical stability of Form CSI

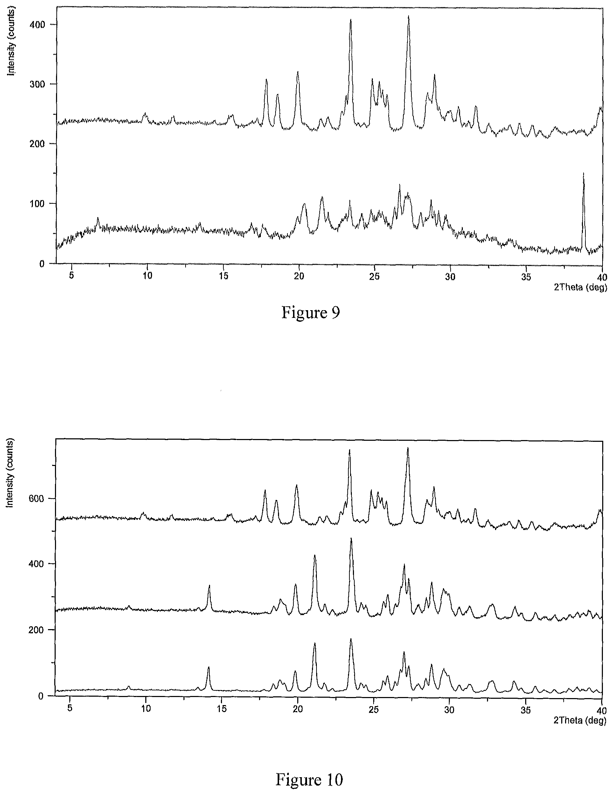

A certain amount of Form CSI of the present disclosure and Form A were weighed and stored under different conditions of 25 °C/60%RH, 40 °C/75%RH and 60 °C/75% RH. Chemical purity and crystalline form were checked by HPLC and XRPD, respectively. The results are shown in Table 8 and Table 9, and the XRPD overlay of Form CSI and Form A before and after storage at different conditions are shown in Figure 8 and Figure 9.

**Table 8**

| Initial form | Condition | Time | Form | Purity |
|---|---|---|---|---|
| Form CSI | initi al | - | Form CSI | 99.91 % |
| | 25 °C/60%RH (sealed) | 6 months | Form CSI | 99.89% |
| | 25 °C/60%RH (open) | 6 months | Form CSI | 99.94% |
| | 40 °C/75% RH (sealed) | 6 months | Form CSI | 99.78% |
| | 40 °C/75% RH (open) | 6 months | Form CSI | N/A |

**Table 9**

| Initial form | Condition | Time | Form |
|---|---|---|---|
| Form CSI | initial | - | Form CSI |
| | 60 °C/75% RH (sealed) | 2 months | Form CSI |
| | 60 °C/75% RH (open) | 2 months | Form CSI |
| Form A | 60 °C/75% RH (open) | 1 month | Crystalline form change |

The results show that Form CSI is stable for at least six months at 25 °C/60% RH (open and sealed) and 40 °C/75% RH (open and sealed) conditions. Form CSI is physically stable for at least two months at 60 °C/75% RH (open and sealed) conditions. The crystalline form of Form A changes after storing at 60 °C/75% RH (open) condition for one month.

Consequently, Form CSI has good physical and chemical stability under low temperature, long-term, accelerated and stress conditions. Form CSI has better stability compared with Form A.

### Example 6 Thermodynamic stability of Form CSI

Form CSI of the present disclosure, Form A, and certain volume of solvents were mixed for 10 seconds, and then small portions of solid were separated to characterize the crystalline form by XRPD. The suspensions were stirred at certain temperature for two days. The obtained solids were separated, and the crystalline form was checked by XRPD. The results are shown in Table 10. The XRPD overlay of sample 4 before and after stirring is shown in Figure 10.

**Table 10**

| Sample No. | Mass of Form CSI (mg) | Mass of Form A (mg) | Solvent (v/v) | Temperature | Crystalline form after mixing for 10 seconds | Crystalline form after stirring for 2 days |
|---|---|---|---|---|---|---|
| 1 | 4.1 | 18.2 | Ethanol | 50 °C | Form A + Form CSI | Form CSI |
| 2 | 3.5 | 15.0 | | Room temperature | Form A + Form CSI | |
| 3 | 4.6 | 24.1 | Ethyl acetate | 50 °C | Form A + Form CSI | |
| 4 | 5.3 | 12.6 | | Room temperature | Form A + Form CSI | |
| 5 | 8.8 | 14.1 | Water/ethyl acetate (1:9) | Room temperature | Form A + Form CSI | |

The results indicate that Form CSI is thermodynamically more stable compared with Form A.

### Example 7 Mechanical stability of Form CSI

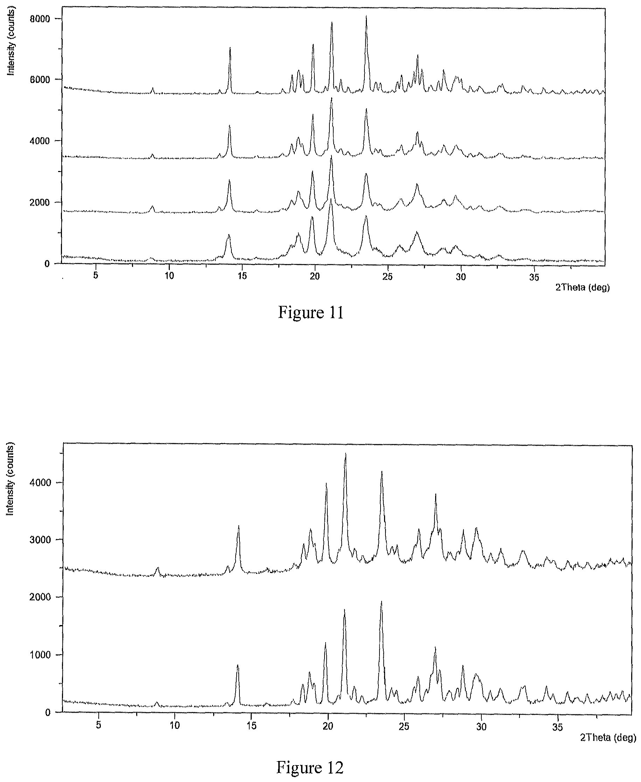

30 mg of Form CSI was weighed and compressed into pellets under different pressures with the dies of ϕ6 mm round tooling manually. Crystalline form before and after tableting were checked by XRPD. The results are shown in Table 11 and Figure 11.

**Table 11**

| Before tableting | Pressure | After tableting |
|---|---|---|
| Form CSI | 3kN | Form CSI |
| | 7kN | Form CSI |
| | 14kN | Form CSI |

The results show that Form CSI has good stability under different pressures.

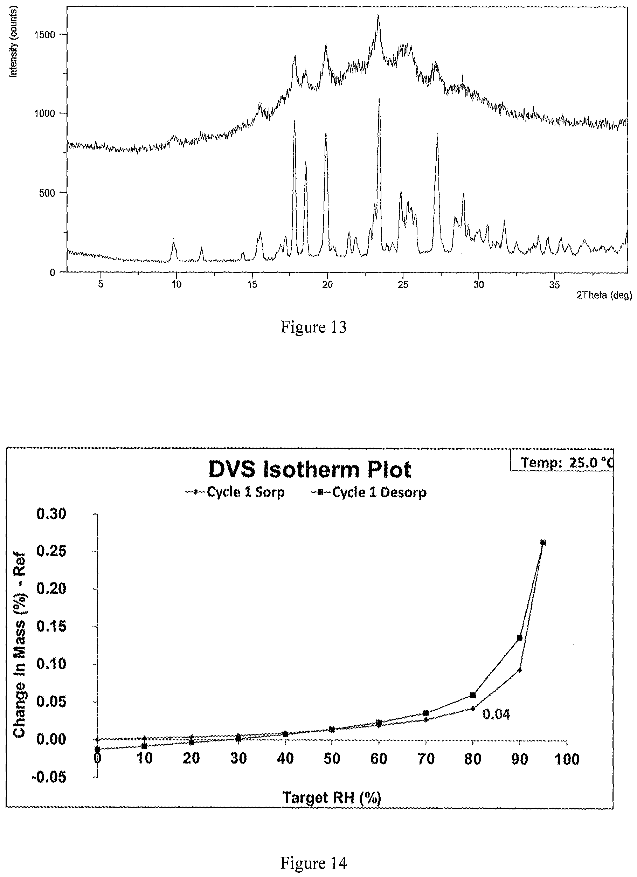

Form CSI and Form A were ground manually for 5 minutes in mortars. Crystalline form before and after grinding were checked by XRPD. The results are listed in Table 12. The XRPD before and after grinding are shown in Figure 12 and Figure 13, respectively.

**Table 12**

| Before grinding | After grinding |
|---|---|
| Form CSI | Form CSI |
| Form A | Mostly transformed to amorphous |

The results show that Form CSI shows better stability under grinding compared with Form A.

### Example 8 Hygroscopicity of Form CSI

Dynamic vapor sorption (DVS) was applied to test hygroscopicity of Form CSI with a certain amount of sample. The weight gains at each relative humidity were recorded in a cycle of 0-95%-0 RH at room temperature. The result is listed in Table 13. The DVS plot of Form CSI is shown in Figure 14.

**Table 13**

| Crystalline form | Weight gain under 80%RH |
|---|---|
| Form CSI | 0.04% |

Description and definition of hygroscopicity (general notice 9103 drug hygroscopicity test guidelines in 2015 edition of Chinese Pharmacopoeia, test at 25 °C±1 °C, 80% RH. The definition of hygroscopicity in the 9^{th} European Pharmacopoeia 5.11 is in consistent with the Chinese Pharmacopoeia.).
- deliquescent: Sufficient water is absorbed to form a solution.
- very hygroscopic: Increase in mass is equal to or greater than 15%.
- hygroscopic: Increase in mass is less than 15% and equal to or greater than 2%.
- slightly hygroscopic: Increase in mass is less than 2% and equal to or greater than 0.2%.
- non hygroscopic or almost non hygroscopic: Increase in mass is less than 0.2%. Weight gain of Form CSI under 80% RH is 0.04%. Form CSI is non hygroscopic or almost non hygroscopic.

### Example 9 Particle size distribution of Form CSI

About 10-30 mg of Form CSI was added into a glass vial with about 10 mL of Isopar G (containing 0.2% lecithin). The mixture was mixed thoroughly and transferred into the Hydro MV dispersing device. The experiment was started when the obscuration was in appropriate range. The particle size distribution (PSD) was tested after 30 seconds of ultrasonication. The average particle diameter calculated by volume, the diameter at which 10% mass was comprised of smaller particles, the diameter at which 50% mass was comprised of smaller particles, and the diameter at which 90% mass was comprised of smaller particles were obtained. The results are shown in Table 14.

**Table 14**

| Form | Ultrasonication time (s) | MV (µm) | D10 (µm) | D50 (µm) | D90 (µm) |
|---|---|---|---|---|---|
| Form CSI | 0 | 9.97 | 1.01 | 5.31 | 16.3 |
| | 30 | 6.25 | 0.975 | 4.67 | 11.7 |

| | | | | | |
|---|---|---|---|---|---|
| MV: average particle diameter calculated by volume. D10: the size in microns below which 10 percent of the particles reside on a volume basis. D50: the size in microns below which 50 percent of the particles reside on a volume basis, also known as the median diameter. D90: the size in microns below which 90 percent of the particles reside on a volume basis. | | | | | |

The results show that the particle size distribution of Form CSI is uniform.

### Example 10 Density of Form CSI

500 mg of powder was added into a 5-mL measuring cylinder and bulk volume was recorded. Then the powder was tapped 1250 times with a ZS-2E tap density tester to make it in the tightest state and the tapped volume was recorded. The bulk density (po) and tapped density (ρ_{f}) were calculated.

Evaluation of density of Form CSI and Form A is shown in Table 15.

**Table 15**

| Form | Bulk density (g/mL) | Tapped density (g/mL) |
|---|---|---|
| Form A | 0.287 | 0.408 |
| Form CSI | 0.328 | 0.449 |

The results indicate that the density of Form CSI is higher than that of Form A.

### Example 11 Adhesiveness of Form CSI

30 mg of Form CSI and Form A were weighed and then added into the dies of ϕ8mm round tooling, compressed with an ENERPAC manual tablet press at 10 kN and held for 30 s. The material sticking to the punch was calculated. The compression was repeated twice and the cumulative amount, maximum amount and average amount of material sticking to the punch during the compression process were recorded. Detailed experimental results are shown in Table 16.

**Table 16**

| Form | Maximum amount (mg) | Average amount (mg) |
|---|---|---|
| Form A | 0.22 | 0.13 |
| Form CSI | 0.16 | 0.11 |

Test results indicate that the adhesiveness of Form CSI is superior to that of Form A.

### Example 12 Preparation of CSI drug product

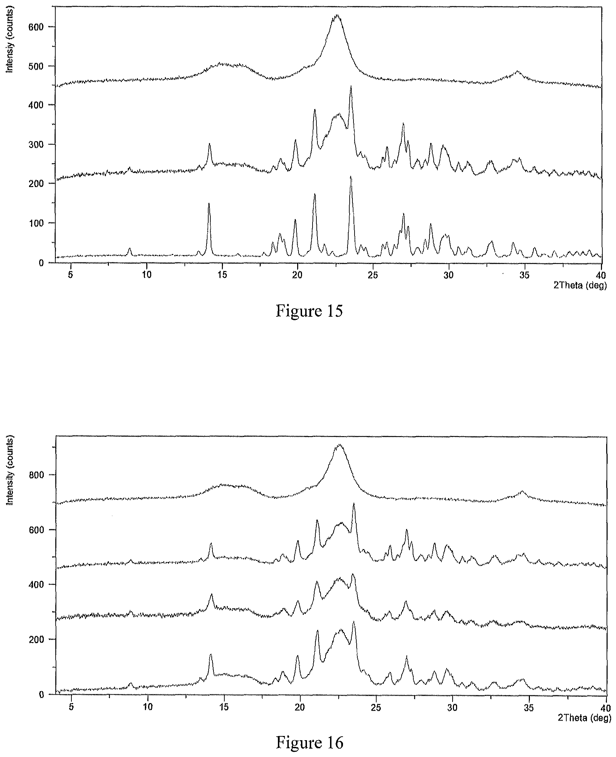

The formulation and preparation process of Form CSI drug product are shown in Table 17 and Table 18, respectively. The XRPD patterns of Form CSI before and after the formulation process are shown in Figure 15.

**Table 17**

| No. | Component | mg/ tablet | % (w/w) | Function |
|---|---|---|---|---|
| 1 | Aprocitentan (free form) | 25.0 | 25.0 | API |
| 2 | Microcrystalline cellulose (Avicel PH102) | 68.5 | 68.5 | Filler |
| 3 | Crospovidone (Polyplasdone XL) | 6.0 | 6.0 | Disintegration |
| 4 | Magnesium stearate (SH-YM-M) | 0.5 | 0.5 | Lubricant |
| Total | | 100.0 | 100.0 | / |

**Table 18**

| Stage | Procedure |
|---|---|
| Pre-blending | According to the formulation, No. 1-4 materials were weighed into a glass vial and blended for 2 mins; |
| Sifting | The pre-blend powder was passed through a 3 5-mesh sieve and put into a glass vial and mixed for 1 min; |
| Simulated dry granulation | The mixture was pressed by a single punch manual tablet press (type: ENERPAC; die: ϕ 20 mm round; tablet weight: 500 mg; pressure: 5 ± 1 KN); |
| Milling | The flakes were pulverized and sieved through a 20-mesh sieve, and blended for 1 min; |
| Compression | The mixture was pressed by a single punch manual tablet press (type: ENERPAC; die: ϕ7 mm round; tablet weight: 100.0 mg; pressure: 5 ± 1 KN); |
| Package | The tablets were packed in 35 cc HDPE bottles with 1g of desiccant inside, 1 tablet/bottle. |

Test results indicate that Form CSI remains unchanged before and after the formulation process. Form CSI has good stability in the formulation process.

### Example 13 Stability of Form CSI in drug product

The tablets containing Form CSI were packed in HDPE bottles and stored under 25 °C/60% RH and 40 °C/75% RH conditions for one month. Crystalline form and impurity of the samples were tested to check the stability of Form CSI drug product. The results indicate that Form CSI drug product can keep stable under 25 °C/60% RH and 40 °C/75% RH for at least one month. The assay of related substances before and after storage are shown in Table 19. The XRPD overlay before and after storage are shown in Figure 16.

**Table 19**

| Form CSI drug product | Purity % | Maximum single impurity % | Total impurity % |
|---|---|---|---|
| Initial | 99.91 | 0.09 | 0.09 |
| 25 °C/60%RH | 99.90 | 0.10 | 0.10 |
| 40 °C/75%RH | 99.87 | 0.13 | 0.13 |

The results indicate that the Form CSI drug product remains unchanged after one month-storage at 25 °C/60%RH and 40 °C/75%RH sealed conditions with 1 g of desiccant, and the related substances do not increase obviously. The chemical stability of Form CSI is good in drug product.

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A crystalline form CSI of Aprocitentan, wherein the X-ray powder diffraction pattern comprises characteristic peaks at 2theta values of 14.2°±0.2°, 21.1°±0.2°and 23.5°±0.2° using CuKa radiation.

2. The crystalline form CSI according to claim 1, wherein the X-ray powder diffraction pattern comprises one or two or three characteristic peaks at 2theta values of 19.9°±0.2°, 28.8°±0.2° and 27.3°±0.2° using CuKa radiation.

3. The crystalline form CSI according to claim 1, wherein the X-ray powder diffraction pattern comprises one or two or three characteristic peaks at 2theta values of 25.9°±0.2°, 28.0°±0.2° and 29.6°±0.2° using CuKa radiation.

4. The crystalline form CSI according to claim 1, wherein the X-ray powder diffraction pattern is as depicted in Figure 1 using CuKa radiation.

5. A process for preparing crystalline form CSI of Aprocitentan according to claim 1, wherein the process comprises:
(1) adding Aprocitentan solid into a mixture of water and an ester, stirring the suspension, centrifuging and drying to obtain crystalline form CSI; or
(2) dispersing Aprocitentan solid and crystalline form CSI seed in an alcohol, an ester or a mixture of water and an ester, stirring the suspension, centrifuging and drying to obtain crystalline form CSI.

6. The process according to claim 5, wherein in method (1), said ester is ethyl acetate, the ratio of water and ethyl acetate of the mixture is 1:20-1:3, said stirring temperature is -15 °C-15 °C, the time of said stirring is more than 10 days; in method (2), said alcohol is ethanol, the said ester is ethyl acetate.

7. The process according to claim 5, wherein in method (2), the temperature of said stirring is -20 °C-70 °C, the time of said stirring is more than half an hour.

8. The process according to claim 6, wherein in method (1), the ratio of said mixture of water and ethyl acetate is 1:9, the temperature of said stirring is 5 °C, the time of said stirring is 15 days.

9. A pharmaceutical composition, wherein said pharmaceutical composition comprises a therapeutically effective amount of crystalline form CSI according to claim 1, and pharmaceutically acceptable carriers or excipients.

10. Crystalline form CSI according to claim 1 for the use of preparing drugs antagonizing dual endothelin receptor.

11. Crystalline form CSI according to claim 1 for preparing drugs treating resistant hypertension.
